# FASCICULE DE BREVET EUROPEEN

(11) **EP 4 291 281 B1**
(45) Date de publication et mention de la délivrance du brevet: **09.04.2025**
(21) Numéro de dépôt: 22708193.2
(22) Date de dépôt: 07.02.2022
(51) Int. Cl.: A61M 37/00

(54) **APPLICATEUR A MICRO-AIGUILLES**
MIKRONADELAPPLIKATOR
MICRONEEDLE APPLICATOR

(30) Priorité: 09.02.2021 FR 2101225
(43) Date de publication de la demande: 20.12.2023
(73) Titulaire: APTAR France SAS, 27110 Le Neubourg (FR)
(72) Inventeur: BIZET, Bruno, 71000 Sancé (FR); CHAMARANDE, Vincent, 71000 Mâcon (FR)
(74) Mandataire: CAPRI
(86) Numéro de dépôt international: PCT/FR2022/050225
(87) Numéro de publication internationale: WO 2022/171953

(56) Documents cités:
- CN-A- 111 672 023
- CN-U- 204 017 140
- KR-A- 20170 119 932
- KR-A- 20200 019 399
- KR-A- 20210 004 357
- US-A1- 2013 345 638
- US-A1- 2016 235 392
- US-A1- 2018 280 675

## Description

La présente invention concerne un applicateur à micro-aiguilles pour appliquer un produit fluide sur la peau et le faire pénétrer dans la couche supérieure de l'épiderme. Le domaine d'application de l'invention est celui de la cosmétique, et non pas celui du tatouage. Le but est de renforcer l'efficacité d'un traitement cosmétique dans la peau, et non pas de colorer la peau.

De manière conventionnelle, ce type d'applicateur cosmétique comprend une face d'application pourvue d'au moins une sortie de produit fluide et de plusieurs micro-aiguilles. Un moteur, souvent électrique, est utilisé pour faire vibrer les micro-aiguilles, seules ou avec la face d'application. Un réservoir de produit fluide est relié à la sortie de produit fluide. Le réservoir peut être intégré ou non à l'applicateur. Lorsqu'il est intégré, il se pose alors la question de son remplissage ou de son remplacement. L'applicateur peut être démontable ou comprendre une fenêtre pour accéder au réservoir.

US 2018/280675 A1 décrit un applicateur à micro-aiguilles ayant plusieurs éléments et étant réutilisable.

D'autre part, un organe d'actionnement est aussi prévu pour acheminer le produit fluide du réservoir de produit fluide à la sortie de produit fluide. Cet organe d'actionnement est souvent celui qui commande aussi le moteur, qui cumule alors une double fonction, à savoir de faire vibrer les micro-aiguilles et d'acheminer le produit fluide du réservoir à la face d'application.

Le but de la présente invention est de proposer un applicateur très simple, aussi bien en termes de conception et d'utilisation, avec une distribution de produit fluide simple et intuitive.

L'invention est définie dans la revendication 1. D'autres aspects sont définis dans les revendications dépendantes 2-15.

Pour atteindre ce but, la présente invention prévoit un applicateur à micro-aiguilles pour appliquer un produit fluide sur la peau et le faire pénétrer dans la peau, l'applicateur définissant un axe longitudinal et comprenant :
- une face d'application pourvue d'au moins une sortie de produit fluide et de plusieurs micro-aiguilles,
- un moteur pour faire vibrer les micro-aiguilles,
- au moins un réservoir de produit fluide relié à la sortie de produit fluide,
- un organe d'actionnement pour acheminer le produit fluide du réservoir de produit fluide à la sortie de produit fluide,
caractérisé en ce qu'il comprend trois modules distincts raccordés axialement l'un à l'autre, à savoir :
- un module moteur abritant le moteur et des accessoires pour faire fonctionner le moteur,
- un module cartouche abritant le ou les réservoir(s) de produit fluide et formant la face d'application,
- un module intermédiaire raccordé au module moteur et raccordé de manière amovible au module cartouche, le module intermédiaire comprenant l'organe d'actionnement et des moyens de transmission pour transmettre les vibrations générées par le moteur du module moteur aux micro-aiguilles du module cartouche.

La distribution de produit fluide est donc gérée manuellement par l'utilisateur qui agit à l'aide d'un doigt sur l'organe d'actionnement du module intermédiaire. On comprend alors que le module cartouche est techniquement très simple, puisqu'il ne comprend aucun composant électrique ou électronique et très peu d'organes mécaniques. Il constitue une cartouche ou recharge remplaçable peu couteuse, que l'utilisateur peut facilement séparer et connecter au module intermédiaire, qui est lui aussi relativement simple, puisqu'il intègre principalement des moyens de transmission mécaniques, à savoir les moyens de transmission de vibrations et des moyens de transmission de poussée permettant de convertir le déplacement de l'organe d'actionnement en poussée sur le réservoir. Le module moteur intègre lui le moteur, son alimentation et sa commande, et constitue de ce fait un module complexe et couteux.

Cette séparation de l'applicateur en trois modules distincts, le premier très simple, bon marché et remplaçable, le second également simple, mais pérenne et le troisième complexe et couteux, permet d'associer divers modèles de module cartouche à un module moteur standard en configurant le module intermédiaire de manière à ce qu'il s'adapte au module moteur standard et au module cartouche particulier. Plus précisément, le module intermédiaire est conçu pour transmettre les vibrations du module moteur aux microaiguilles du module cartouche et distribuer le produit fluide contenu dans le ou les réservoir(s) du module cartouche. Un organe d'actionnement idoine, et son éventuelle transmission de poussée appropriée, sont logés dans le module intermédiaire.

On peut ainsi assimiler l'architecture de l'applicateur à micro-aiguilles de l'invention à une fusée à trois étages, avec un étage supérieur consommable, un étage inférieur technique et un étage intermédiaire de transmission de vibrations et d'actionnement.

Ainsi, un fabricant de produit cosmétique peut choisir ou concevoir un module cartouche particulier sans se soucier du module moteur qui lui sera associé, étant donné que c'est le module intermédiaire, spécialement adapté à la fois au module moteur et au module cartouche particulier, qui va gérer la transmission des vibrations et l'actionnement du ou des réservoirs. Le module intermédiaire permet ainsi de décorréler le module cartouche du module moteur en jouant un rôle d'interface modulaire. Un large choix d'applicateurs à micro-aiguilles peut de ce fait être proposé pour un coût réduit, puisque le module moteur peut être standard.

Avantageusement, le module intermédiaire est également raccordé de manière amovible au module moteur, par exemple par une connexion vissée ou par baïonnette. Les raccords amovibles permettent une manipulation (connexion et déconnexion) sans l'intervention d'un ustensile extérieur.

En variante, le module intermédiaire peut être rapporté et fixé définitivement ou de manière difficilement amovible au module moteur. Dans les deux cas, les deux modules sont initialement séparés et en aucun cas intégrés l'un à autre de manière indissociable.

Avantageusement, le réservoir de produit fluide est à volume variable, le déplacement de l'organe d'actionnement engendrant une diminution de volume du réservoir de produit fluide, de sorte qu'au moins une partie de son contenu est refoulée à chaque actionnement vers la sortie de produit fluide. Ainsi, l'organe d'actionnement agit directement ou indirectement sur le réservoir sans organe intermédiaire actif, comme une pompe ou un moteur. Le module cartouche peut comprendre un unique réservoir qui est entièrement ou de préférence partiellement vidé à chaque actionnement. En variante, le module cartouche peut comprendre plusieurs réservoirs qui sont successivement vidés (partiellement ou de préférence entièrement) à chaque actionnement.

Avantageusement, le réservoir de produit fluide peut comprendre une paroi mobile ou déformable, qui est déplacée en réponse à l'actionnement de l'organe d'actionnement, selon l'axe longitudinal X ou transversalement à l'axe longitudinal X.

Selon une conception préférée, l'applicateur peut présenter une configuration générale de stylo, adapté à être saisi entre le pouce et le majeur avec l'organe d'actionnement actionnable au moyen de l'index. Il peut également être saisi dans la paume de la main et actionné avec le pouce.

Selon un autre aspect de l'invention, le module cartouche peut être raccordé de manière amovible au module intermédiaire par une connexion combinant un mouvement axial et un mouvement rotatif, tel qu'un vissage ou une baïonnette.

Selon un premier mode de réalisation pratique de l'invention, l'organe d'actionnement peut comprendre un poussoir latéral qui déplace axialement une tige d'actionnement qui agit progressivement sur le réservoir pour refouler des doses successives de produit fluide vers la sortie de produit fluide. Ainsi, le déplacement transversal de l'organe d'actionnement a pour effet de déplacer axialement la tige d'actionnement qui va appuyer sur une paroi mobile (paroi déformable ou piston racleur) du réservoir. Chaque actionnement génère un déplacement limité de la tige qui va refouler une dose de produit fluide du réservoir, qui peut contenir une ou plusieurs doses, par exemple quatre. La tige remplit une fonction de transmission de poussée : la poussée transversale sur l'organe d'actionnement est transformée en une poussé axiale de la tige.

Avantageusement, la tige d'actionnement est déplaçable axialement à l'encontre d'un ressort de rappel à partir d'une position de départ rétractée vers plusieurs positions avancées successives, dans lesquelles elle est bloquée en position contre tout retour élastique en arrière par un dispositif de crans d'arrêt. De préférence, le dispositif de crans d'arrêt est pourvu de moyens de débrayage pour permettre au dispositif de crans d'arrêt de libérer la tige d'actionnement, qui retourne alors élastiquement vers sa position de départ rétractée. Avantageusement, les moyens de débrayage sont contraints ou bloqués dans un état inopérant par le raccord du module cartouche sur le module intermédiaire et libérés élastiquement ou non dans un état débrayé par la séparation du module cartouche, les moyens de débrayage comprenant avantageusement un organe d'engagement qui est déplaçable à partir d'un état libre, correspondant à l'état débrayé, vers un état contraint, correspondant à l'état opérant, par une came formée par le module cartouche et qui est déplaçable à partir de l'état contraint vers l'état libre par un ressort de rappel, dès qu'il est désengagé de la came.

Ainsi, dans ce mode de réalisation à réservoir unique, la connexion du module cartouche sur le module intermédiaire a pour effet de neutraliser les moyens de débrayage, qui restent inopérants jusqu'à ce que le module cartouche soit retiré du module intermédiaire. Les moyens de débrayage, associés au retrait du module cartouche, remplissent une fonction de remise à zéro ou de « reset » pour la tige d'actionnement, quelle que soit sa position avancée, qui revient alors automatiquement dans sa position de départ rétractée : l'utilisateur n'a pas besoin d'effectuer une quelconque manipulation pour ramener la tige en position de départ.

La coopération entre la came du module cartouche et l'organe d'engagement des moyens de débrayage remplit également une fonction d'authenticité du module cartouche, car cette coopération est comparable à celle qui existe entre une serrure et une clé. Plus généralement, on peut ainsi dire que le module cartouche et le module intermédiaire forment ensemble des moyens d'identification propre qui garantissent l'authenticité du module cartouche. Ces moyens d'identification propre peuvent être constitués par la coopération entre la came du module cartouche et l'organe d'engagement des moyens de débrayage, mais peuvent également être constitués par n'importe quel engagement coopératif ou complémentaire entre ces deux modules.

Bien entendu, en l'absence d'organe d'engagement et de came, c'est à l'utilisateur d'actionner les moyens de débrayage pour ramener la tige à zéro.

Il est à noter que la tige d'actionnement à déplacement axial, associée à un dispositif de crans d'arrêt et avantageusement à des moyens de débrayage est une caractéristique qui peut être mise en œuvre dans un applicateur ne comprenant que deux modules, à savoir un module moteur intégrant le moteur, l'organe d'actionnement, la tige, le dispositif de crans d'arrêt et avantageusement des moyens de débrayage, et un module cartouche identique ou similaire à celui déjà décrit, qui peut servir à neutraliser/libérer les moyens de débrayage lors de son montage/retrait. En d'autres termes, une protection pourrait être recherchée pour ces caractéristiques dans un applicateur en deux modules, et même un applicateur en un seul module, avec le réservoir remplaçable.

Selon un deuxième mode de réalisation pratique de l'invention, le module cartouche peut comprendre un barillet rotatif supportant plusieurs réservoirs de produit fluide, qui peuvent ainsi être amenés à tour de rôle dans une position de vidage, le module intermédiaire comprend un poussoir latéral qui agit sur le réservoir situé en position de vidage pour refouler son contenu vers la sortie de produit fluide.

Selon une première variante, le poussoir latéral peut déplacer axialement une tige d'actionnement à l'encontre d'un ressort de rappel à partir d'une position de départ vers une position étendue correspondant au vidage maximal du réservoir. La tige d'actionnement remplit une fonction de transmission de poussée : la poussée transversale sur le poussoir est transformée en une poussé axiale de la tige.

Selon une seconde variante, le poussoir latéral peut agir directement sur le réservoir à travers une fenêtre latérale du barillet. Avantageusement, le module intermédiaire peut comprendre des moyens d'actionnement pour entrainer le barillet du module cartouche en rotation. De préférence, la face d'application du module cartouche peut être bloquée en rotation par le module intermédiaire, chaque réservoir comprenant un organe d'obturation amovible qui est retiré avant son arrivée en position de vidage par un organe d'ouverture solidaire de la face d'application. Les réservoirs peuvent par exemple se présenter sous la forme de petites fioles souples avec une tête d'obturation qui est coupée par une lame fixe juste avant de gagner la position de vidage, dans laquelle le poussoir va l'écraser et refouler son contenu vers la sortie au niveau de la face d'application.

Il est à noter que le barillet rotatif à plusieurs réservoirs à actionnement latéral ou axial est une caractéristique qui peut être mise en œuvre dans un applicateur ne comprenant que deux modules, à savoir un module moteur intégrant le moteur et le poussoir latéral, et un module cartouche à barillet identique ou similaire à celui déjà décrit. En d'autres termes, une protection pourrait être recherchée pour ces caractéristiques dans un applicateur en deux modules, et même un applicateur en un seul module, avec les réservoirs remplaçables.

Selon un autre aspect, la face d'application peut comprendre une platine dotée de micro-aiguilles et traversée par la sortie de produit fluide. La sortie de produit fluide peut également se faire en périphérie de la face d'application.

Avantageusement, l'applicateur peut en outre comprendre des moyens d'ajustement de la profondeur de pénétration des micro-aiguilles, agissant avantageusement sur la position axiale du moteur dans le premier module. En effet, il est parfois utile de faire pénétrer plus ou moins les micro-aiguilles en fonction du produit cosmétique, du traitement souhaité ou de la qualité et la nature de la peau.

L'applicateur peut également comprendre des moyens de commande du moteur, qui le désactivent automatiquement lorsque les modules ne sont pas connectés. Il s'agit d'une sécurité d'utilisation.

L'invention réside dans le fait de segmenter le dispositif applicateur à microaiguilles en trois modules, unités ou sous-ensembles, avec un module standard, un module sur mesure jetable et un module médian qui fait la transmission entre les deux autres modules et qui intègre les moyens pour refouler du produit fluide hors du ou des réservoirs vers la face d'application du module jetable, qui comprend des microaiguilles.

L'invention sera maintenant plus amplement décrite en référence aux dessins joints qui donnent, à titre d'exemples non limitatifs, plusieurs modes de réalisation de l'invention.

Sur les figures :
La figure 1 est une vue schématique en perspective localement découpée d'un applicateur à micro-aiguille selon une première forme de réalisation de l'invention,
La figure 2 est une vue en coupe transversale verticale axiale à travers l'applicateur de la figure 1,
La figure 3 est une vue similaire à celle de la figure 2 avec l'applicateur dans un état démonté,
La figure 4 est une vue similaire des figures 2 et 3 avec l'applicateur déjà actionné,
La figure 5 est une vue similaire à celles des figures 2 à 4 avec l'applicateur dans un état débrayé,
Les figures 6a et 6b sont des représentations très schématiques visant à illustrer une forme de réalisation des moyens de débrayage et de leur actionnement,
Les figures 7a et 7b représentent un autre mode de réalisation pour les moyens de débrayage et leur actionnement,
La figure 8 est une vue en coupe transversale verticale à travers les modules cartouche et intermédiaire d'un applicateur selon un deuxième mode de réalisation de l'invention,
La figure 9a et 9b sont des vues similaires à celle de la figure 8, respectivement en positions actionnée et de retour,
Les figures 10a et 10b sont des vues schématiques en coupe transversale verticale à travers un applicateur de l'invention selon un troisième mode de réalisation, respectivement à l'état de repos et à l'état actionné, et
La figure 11 est une vue en perspective de l'applicateur des figures 10a et 10b à l'état démonté.

L'applicateur de l'invention est purement cosmétique, voire dermatologique, à l'exclusion du tatouage. Il associe deux moyens de traitement, à savoir la distribution d'un produit cosmétique, qui peut être une crème, une pommade, une lotion, un sérum, etc., et la perforation du stratum corneum pour atteindre l'épiderme, sans toucher le derme, au moyen de micro-aiguilles. Selon la nature du produit cosmétique et le résultat recherché, le produit cosmétique est appliqué avant, en même temps ou après la micro-perforation. L'applicateur de l'invention est plutôt à usage domestique, en ce sens que l'utilisateur de l'applicateur va l'utiliser sur lui-même. Il peut toutefois s'utiliser de manière professionnelle.

Dans les trois modes de réalisation de l'invention illustrés sur les figures, l'applicateur de l'invention comprend trois modules distincts, à savoir un premier module moteur M, un second module cartouche C1, C2, C3 et un troisième module intermédiaire I1, I2, I3, monté ou interposé entre les modules moteur M et cartouche C1, C2, C3 : le module intermédiaire I1, I2, I3 fait ainsi la jonction entre les modules moteur M et cartouche C1, C2, C3. Les trois modules sont disposés selon un axe longitudinal X. Le module intermédiaire I1, I2, I3 est raccordé, avantageusement de manière amovible au module moteur M. Une connexion définitive entre ces deux modules est envisageable. Le module intermédiaire I1, I2, I3 est raccordé, avantageusement de manière amovible au module cartouche C1, C2, C3. Une connexion définitive est envisageable dans certains cas. De préférence, les modules sont connectables et déconnectables de manière simple et rapide, par l'utilisateur lui-même, par exemple à l'aide de ses deux mains en imprimant un couple et/ou une poussée/traction entre deux modules. Lorsque les trois modules sont assemblés, l'applicateur présente une configuration générale en forme de stylo selon l'axe longitudinal X. L'applicateur peut d'ailleurs être saisi de la même manière qu'un stylo.

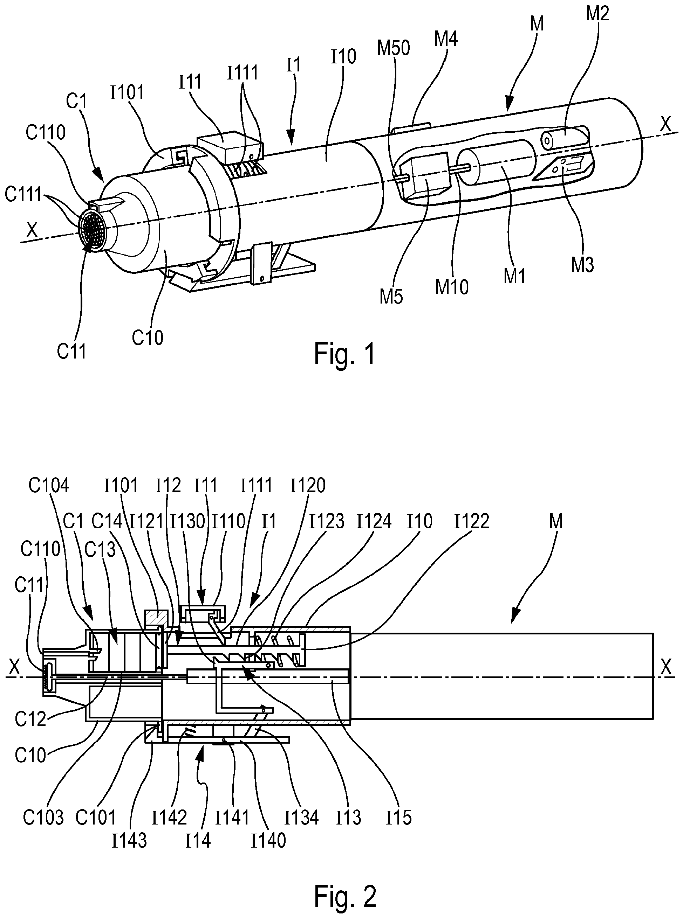

Le premier module moteur M peut être commun aux deux modes de réalisation. Il ne sera pas décrit en détail, car il n'est pas critique pour l'invention. Il peut s'agir d'un module standard du commerce. Sur la figure 1, on peut voir que le module moteur M renferme un moteur M1, qui est de préférence un moteur électrique. Il peut s'agir d'un petit moteur rotatif qui entraine un arbre M10 en rotation sur lui-même. On peut aussi prévoir un moteur à électroaimant, un moteur linéaire ou un piézoélectrique. Le moteur M1 est alimenté par une batterie M2 et commandé par une électronique M3, qui va gérer la vitesse de rotation de l'arbre M10, les séquences et les durées d'activation du moteur, etc. Un bouton d'activation externe M21 permet à l'utilisateur de mettre l'applicateur sous tension. L'extrémité libre de l'arbre M10 est coiffé d'un boitier M5, permettant de transformer la rotation de l'arbre M10 en une oscillation axiale vibratoire, transmise par un arbre de sortie M50, destiné à se raccorder au module intermédiaire.

Bien entendu, il ne s'agit là que d'un mode de réalisation non limitatif : n'importe quel module moteur M adapté à générer une vibration axiale peut être mis en œuvre dans le cadre de la présente invention, étant donné que le module moteur M n'est pas critique pour l'invention.

On se réfèrera maintenant à la figure 2 pour décrire en détail la structure du module cartouche C1 et du module intermédiaire I1 d'un applicateur selon un premier mode de réalisation de l'invention.

Le module cartouche C1 comprend un corps de cartouche C10 qui comprend à une de ses extrémités une collerette de connexion C101 et à son autre extrémité un orifice de sortie de produit fluide C110. Le module cartouche C1 comprend une face d'application C11 qui est pourvue de plusieurs micro-aiguilles C111. Cette face d'application C11 est disposée de manière adjacente à la sortie de produit fluide C110. La face d'application C11 est reliée à une tige de transmission de vibration C12 qui traverse le corps de cartouche C10. La cartouche C1 contient également un réservoir de produit fluide C13 qui est disposé de manière coulissante dans une chambre C103 formée par le corps de cartouche C10. Au fond de cette chambre C103, le corps de cartouche C10 forme un pointeau de perforation C104 qui communique directement par un conduit à l'orifice de sortie de produit fluide C110. Le réservoir de produit fluide C13 comprend un piston pousseur C14 qui est monté de manière coulissante à l'intérieur du réservoir de manière à en faire varier son volume utile. Le réservoir de produit fluide C13 peut ainsi se présenter sous la forme d'un cylindre pourvu à une de ses extrémités du piston pousseur C14 et à son autre extrémité d'une membrane perçable C131 destinée à être percée par le pointeau de perçage C103 du corps de cartouche C10. Le réservoir de produit fluide C13 peut d'ailleurs être un composant qui peut être retiré du corps C10 après usage et remplacé par un réservoir neuf de remplacement.

Le module cartouche C1 est connecté de manière amovible, notamment par rotation (baïonnette ou vissage), au module intermédiaire I1 qui comprend à cet effet une couronne de connexion I101 dans laquelle la collerette C101 peut être reçue de manière stable après rotation. Cette couronne de connexion I101 est formée par un corps I10 qui est également connecté, avantageusement de manière amovible au module moteur M. La connexion peut se faire par vissage, baïonnette ou encliquetage. Une connexion définitive est également possible. Le module intermédiaire I1 comprend un organe d'actionnement 111 qui peut se présenter sous la forme d'un poussoir latéral, qui se déplace donc perpendiculairement à l'axe X. Cet organe d'actionnement 111 peut faire saillie par rapport au corps I1 à travers une fenêtre latérale du corps I10. L'organe d'actionnement 111 comprend des pattes de poussée 1111 dont la fonction sera donnée ci-après. Ces pattes 1111 s'étendent de manière inclinée vers l'intérieur du corps I10. Le module intermédiaire I1 comprend également une tige d'actionnement I12 qui s'étend parallèlement à l'axe X. Cette tige 112 comprend une platine de poussée 1121 destinée à venir en contact avec le piston pousseur C14 du réservoir C13 du module cartouche C1. La tige I12 comprend sur sa longueur plusieurs dents I123, ainsi que des tétons de poussée (non représentés). L'extrémité opposée à la platine de poussée I12 forme un talon I122 sur lequel prend appui un ressort de rappel I124. Ainsi, la tige d'actionnement I12 est sollicitée en éloignement du piston pousseur C14 par le ressort de rappel I124. Le déplacement de la tige d'actionnement I12 en direction du piston pousseur C14 est généré par l'enfoncement de l'organe d'actionnement I11 dont les pattes de poussée I111 viennent en prise avec les ergots de poussée (non représentés) de la tige d'actionnement I12. L'orientation inclinée des pattes de poussée I111 a pour effet de déplacer la tige d'actionnement 112 en direction du piston pousseur C14.

Le module intermédiaire I10 comprend également un dispositif à cran d'arrêt 113 qui comprend un cran d'arrêt 1130 destiné à venir successivement en prise avec les dents 1123 de la tige d'actionnement 112. En d'autres termes, le dispositif à cran d'arrêt 113 permet à la tige d'actionnement 112 de maintenir sa position après chaque actionnement de l'organe d'actionnement 111. Sans ce dispositif à cran d'arrêt I13, la tige I12 reviendrait à chaque fois dans sa position d'origine, sollicitée par le ressort de rappel I124. Le dispositif à cran d'arrêt 113 permet ainsi de neutraliser la sollicitation du ressort 1124.

Le module intermédiaire I1 comprend également des moyens de débrayage I14 qui permettent de débrayer le dispositif de cran d'arrêt I13 de la tige d'actionnement I12. Ces moyens de débrayage I14 peuvent par exemple comprendre une plaque 140 qui est montée pivotante sur un axe I141 solidaire du corps I10. Cette plaque 140 est également reliée au dispositif de cran d'arrêt par une biellette 1134. A son extrémité, la plaque 1140 comprend un organe d'engagement I143 qui vient en prise avec la couronne I101. Enfin, un ressort I142 sollicite la plaque I140 de telle manière à désengager l'organe d'engagement 143 de la couronne I101, le fonctionnement de ces moyens de débrayage sera expliqué ci-après.

Le module intermédiaire I1 comprend également une barre de transmission de vibrations I15 qui relie l'arbre M50 du module moteur M à la tige de transmission C12 du module cartouche C1.

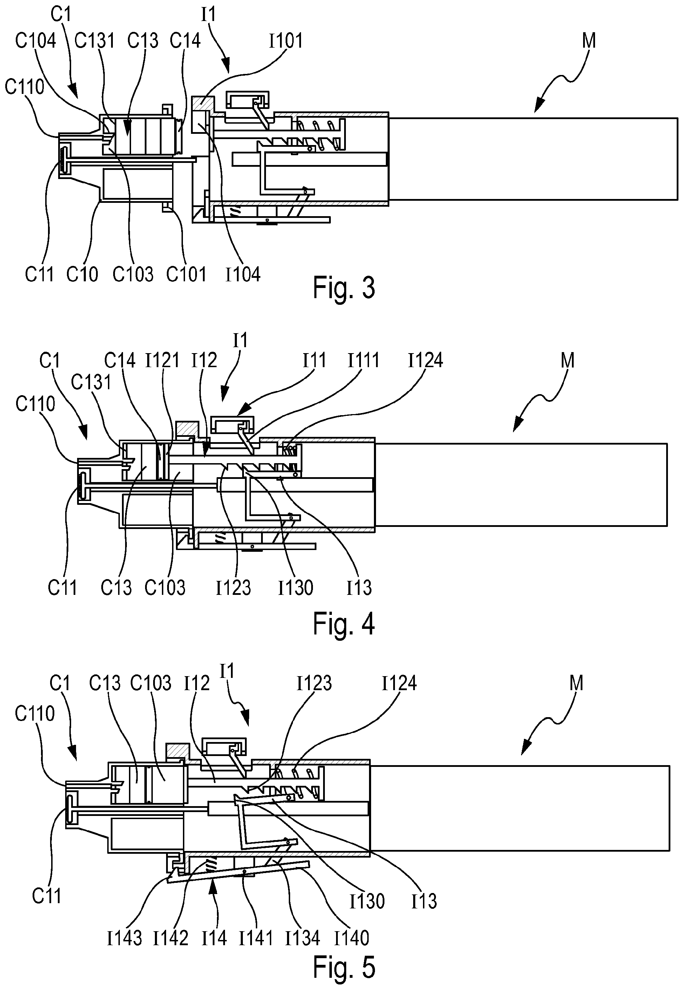

Sur la figure 3, on voit l'applicateur avec le module cartouche C1 déconnecté du module intermédiaire I1. La collerette de connexion C11 est désengagée de la couronne de connexion I101. On pourra alors remarquer que le réservoir C13 n'est pas complètement engagé à l'intérieur de la chambre C103 du corps C10 : le pointeau de percement 104 n'a pas encore pénétré la membrane C131 du réservoir C13 de sorte que le piston pousseur C14 fait saillie hors du corps C10. Le module cartouche est dans sa position initiale avant utilisation, dans laquelle le réservoir C13 est obturé hermétiquement. Lors de la connexion du module cartouche C1 sur le module intermédiaire I1, une patte de poussée I104 formée par le corps I10 va pousser le réservoir C13 en direction du pointeau de perçage 104 de manière à performer la membrane C131 du réservoir C13, qui se trouve alors dans sa position d'utilisation. Dès lors, il suffit à l'utilisateur d'imprimer un mouvement de rotation au module cartouche C1 pour engager sa collerette C101 dans la couronne de connexion I101 du module intermédiaire I1. Une fois connectée, la platine de poussée I121 de la tige d'actionnement I12 vient en contact légèrement appuyé ou non contre le piston pousseur C14 du réservoir C13. Le module intermédiaire I1 est dans sa configuration de départ également représentée sur la figure 2.

L'utilisateur peut alors exercer une poussée sur l'organe d'actionnement I11 de manière à l'enfoncer dans le corps I10 du module intermédiaire I1. Ce faisant, les pattes de poussée I111 vont venir en prise avec les ergots de poussée (non représentés) de la tige d'actionnement I12 qui est alors déplacée axialement contre le piston pousseur C14. En fin d'actionnement, une dent 1123 de la tige 112 va venir en prise avec le cran I130 du dispositif à cran d'arrêt I13. Après deux actionnements successifs, l'applicateur se trouve dans la configuration représentée sur la figure 4. Deux doses de produit fluide ont été distribuées à travers la sortie de produit fluide C110 située à proximité de la face d'application C11 dotée des micro-aiguilles C111. On peut remarquer sur la figure 4 qu'il reste encore deux doses de produit fluide à distribuer. Après chaque dose distribuée, une dent I123 de la tige 112 va venir en prise avec le cran 1130 du dispositif à cran d'arrêt 113.

Grâce aux moyens de débrayage I14, l'utilisateur peut à tout moment renvoyer la tige d'actionnement I12 dans sa position initiale. Ceci est représenté sur la figure 5. On peut y voir que la plaque 1140 est pivotée autour de son axe I141 de manière à désengager son organe d'engagement I143 de la couronne 1101. Ce désengagement est d'ailleurs favorisé par le ressort I142. Pour atteindre cette position, il suffit à l'utilisateur d'appuyer sur la plaque I140 à droite de l'axe de pivotement I141. La plaque I140 va ainsi déplacer la biellette I134 qui va faire basculer le dispositif de cran d'arrêt I13 de manière à désengager son cran 1130 de la dent I23 de la tige C12 avec laquelle il était en prise. On peut voir sur la figure 5 que le cran 1130 est en éloignement de la première dent 1123 et que la tige 112 a regagné sa position initiale sous l'action du ressort de rappel 1124.

Dans ce mode de réalisation, le module intermédiaire I1 permet donc d'agir successivement sur un réservoir de produit fluide C13 pour délivrer des doses successives de produit fluide. Le dispositif à cran d'arrêt I13 permet de maintenir la position de la tige I12 après chaque actionnement et les moyens de débrayage 114 permettent de ramener la tige 112 dans sa position initiale. L'actionnement de moyens de débrayage est ici manuel, en ce sens que l'utilisateur doit agir sur la plaque 140 pour débrayer le dispositif de cran d'arrêt I13 de la tige d'actionnement 112.

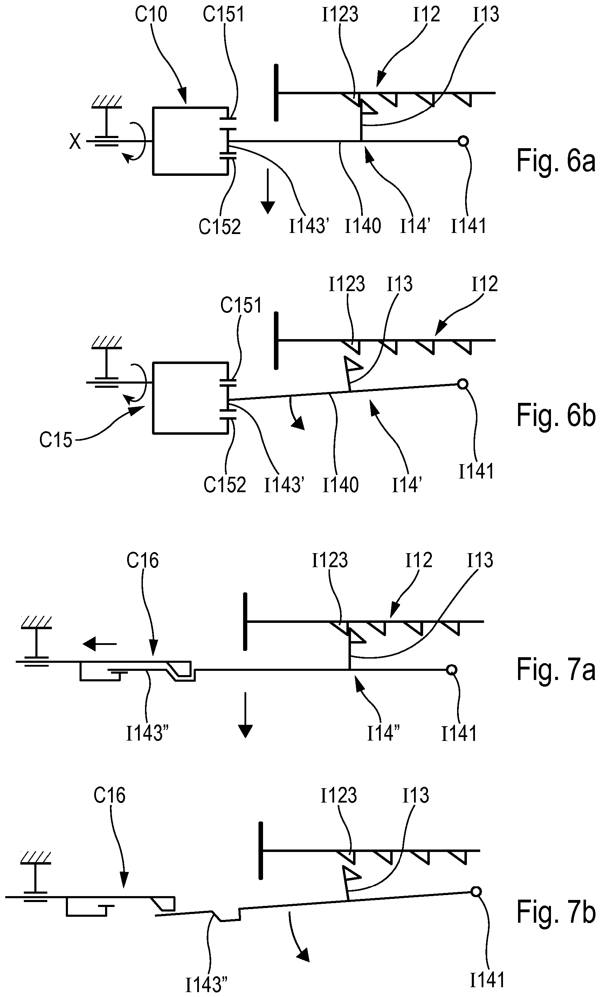

En se référant aux figures 6a et 6b, on peut voir très schématique de quelle manière l'actionnement des moyens de débrayage peut être automatique. La tige de poussée 112 est schématiquement représentée avec sa platine de poussée et ses dents I123. Le dispositif de cran d'arrêt I13 est également représenté de manière très schématisée. Les moyens de débrayage I14' comprennent également une plaque ou une tringle I140 montée pivotante autour d'un axe I141. A son autre extrémité, les moyens de débrayage I14' comprennent un organe d'engagement I143' qui est en prise entre deux cames C151 et C152 formées par le corps C10 du module cartouche C1. Les cames C151 et C152 sont inclinées ou excentrées, de manière à pouvoir entraîner l'organe d'engagement I143' en déplacement autour de l'axe de pivotement I141. La figure 6a représente les moyens de débrayage I14' dans un état opérant, avec le dispositif à cran d'arrêt I13 en prise avec une dent 1123 de la tige d'actionnement I12. Cette position opérante est atteinte lorsque le module cartouche C1 est connecté au module intermédiaire I1.

Sur la figure 6b, on peut voir que la came C151 a entraîné la plaque ou tringle I140 vers le bas par pivotement autour de l'axe de pivotement 1141. Le dispositif à cran d'arrêt 113 est alors désengagé de la dent 1123 de la tige I12. Cette configuration débrayée est atteinte lorsque le module cartouche C1 a été entraîné en rotation par rapport au module intermédiaire I1 jusqu'à ce qu'il puisse être déconnecté. La plaque ou tringle 1140 peut être maintenue dans la position débrayée de la figure 6b par un ressort, comparable au ressort I142 des figures précédentes.

A travers ce mode de réalisation très schématique, on peut comprendre que les moyens de débrayage I14' peuvent être actionnés de manière automatique, simplement par la connexion/déconnexion du module cartouche C1 sur le module intermédiaire I1, à l'aide d'une rotation du module cartouche C1 par rapport au module intermédiaire I1. Ainsi, l'utilisateur n'a même pas à se soucier du retour de la tige d'actionnement I12 vers sa position initiale, puisque la séparation du module cartouche C1 du module intermédiaire I1 entraîne automatiquement l'actionnement des moyens de débrayage qui permettent de ramener la tige 112 dans sa position rétractée de repos.

Les figures 7a et 7b sont des vues extrêmement schématiques visant à faire comprendre que l'actionnement des moyens de débrayage peut également être obtenu par un déplacement purement axial du module cartouche C1 par rapport au module intermédiaire I2. Les moyens de débrayage 114" comprennent un organe d'engagement 1143" en prise avec un logement C16 formé par le module cartouche C1, lorsque le module cartouche C1 est connecté au module intermédiaire I1. Le dispositif à cran d'arrêt 113 est alors en prise avec une dent 1123 de la tige d'actionnement I12.

Dès que l'on déconnecte le module cartouche C1 du module intermédiaire I1, l'organe d'engagement I143" est désengagé du logement C13 et les moyens de débrayage peuvent alors pivoter autour de l'axe I141, de manière à désengager le dispositif à cran d'arrêt I13 de la dent I123. Ceci est représenté sur la figure 7b.

Cet exemple schématique vise à faire comprendre que l'actionnement des moyens de débrayage peut également être réalisé par un déplacement translatif du module cartouche C1 par rapport au module intermédiaire I1.

Ce module intermédiaire I1, avec son organe d'actionnement latéral I11, sa tige d'actionnement 112 à déplacement axial, son dispositif de cran d'arrêt I13 et ses moyens de débrayage manuel I14 ou automatique I14' ou I14", est de préférence connecté de manière amovible à la fois au module moteur M et au module cartouche C1. Toutefois, on peut imaginer des modes de réalisation dans lesquels ce module intermédiaire I1 peut être intégré au module moteur M et/ou au module cartouche C1. On peut même imaginer un applicateur monobloc avec la possibilité de remplacer le réservoir C13 à travers une fenêtre d'accès. Tout particulièrement, les moyens de débrayage constituent une caractéristique qui peut être mise en œuvre dans n'importe quel applicateur comprenant un réservoir à doses multiples.

Les deux prochains modes de réalisation qui vont être décrits ont plusieurs caractéristiques en commun. Tout d'abord, le module intermédiaire comprend un organe de poussée qui agit sur le réservoir du module cartouche de manière à revenir à chaque fois dans sa position initiale de départ. Il n'y a donc pas d'avancée successive comme dans le premier mode de réalisation de l'invention. Quant au module cartouche, il intègre un barillet rotatif chargé avec plusieurs réservoirs : l'utilisateur peut faire tourner le barillet pour ramener un réservoir au droit de l'organe de poussée de manière à ce que le réservoir puisse être majoritairement ou de préférence totalement vidé. Il suffit ensuite à l'utilisateur de faire tourner le barillet rotatif pour amener le prochain réservoir au droit de l'organe de poussée. On verra que l'entraînement en rotation du barillet rotatif peut être effectué directement au niveau du module cartouche ou encore au niveau du module intermédiaire. Bien entendu, le module intermédiaire comprend des moyens de transmission de vibration permettant de transmettre les vibrations générées par le module moteur M à la face d'application pourvue de micro-aiguilles du module cartouche. Également, le module moteur M peut être raccordé de manière amovible au module intermédiaire, tout comme dans le premier mode de réalisation de l'invention. De même, le module intermédiaire peut être raccordé de manière amovible par un mouvement de rotation ou un simple mouvement de translation axiale au module cartouche.

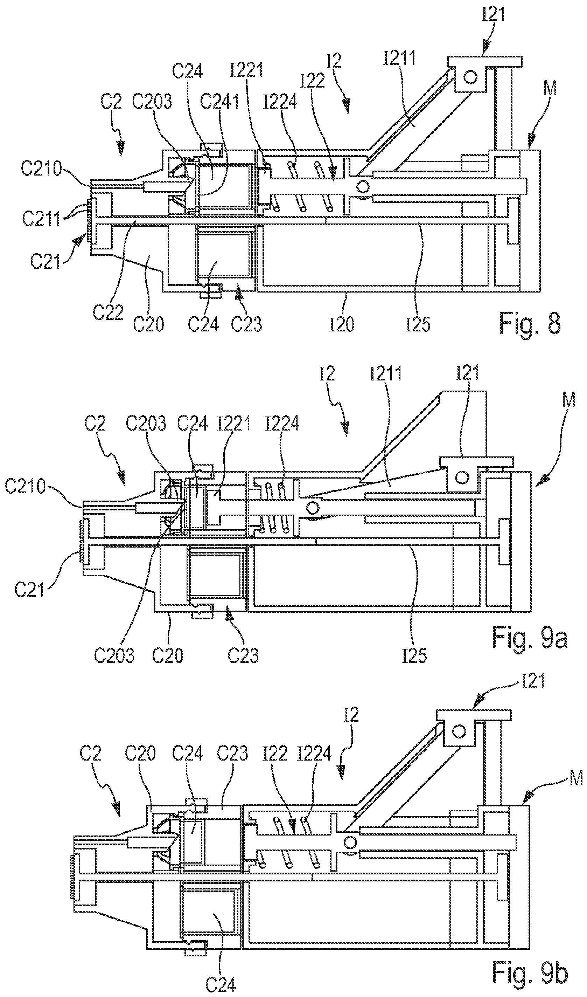

On se référera maintenant aux figures 8, 9a et 9b pour décrire le second mode de réalisation de l'invention. Le module moteur M, qui n'est que très schématiquement représenté, peut être identique ou similaire à celui du premier mode de réalisation. Le module intermédiaire I2 comprend un corps I20 qui est traversé par un organe de transmission de vibration I25 qui s'étend sensiblement ou parfaitement axialement. Le corps I20 contient une tige d'actionnement I22 qui se déplace axialement. Cette tige d'actionnement I22 est sollicitée par un ressort I224 en direction du module moteur M. La tige I22 comprend une platine de poussée I221 destinée à venir en contact d'un des réservoirs du module cartouche, comme on le verra ci-après. La tige I22 est reliée à un organe d'actionnement I21 par une biellette I211. L'organe d'actionnement I21, comme dans le premier mode de réalisation, est déplaçable perpendiculairement à l'axe de l'applicateur. La biellette I211 sert à transformer le déplacement transversal ou latéral de l'organe d'actionnement I21 en un déplacement purement axial de la tige d'actionnement I22. L'enfoncement de l'organe d'actionnement I21 dans le corps I20 s'effectue à l'encontre de la force exercée par le ressort de rappel I224 qui permet de ramener la tige I22 ainsi que l'organe d'actionnement I21 dans sa position de départ étendue, tel que représenté sur les figures 8 et 9b. Dans sa position complètement enfoncée, l'organe d'actionnement I21 se trouve dans la configuration représentée sur la figure 9a. Ainsi, à chaque poussée sur l'organe d'actionnement I21, la tige d'actionnement I22 est déplacée à partir d'une position initiale de départ (figure 8 ou 9b) dans une position étendue maximale (figure 9a), puis retourne sous l'action du ressort de rappel I224 dans sa position initiale de départ. Il n'y a donc pas de position intermédiaire comme dans le premier mode de réalisation de l'invention. Le mécanisme du module intermédiaire I2 est donc plus simple que celui du module intermédiaire I1 du premier mode de réalisation.

Le module cartouche C2 comprend un corps C20 qui est fixe par rapport au corps I1 du module intermédiaire I2 une fois le module cartouche C2 en place sur le module intermédiaire I2. Ce module cartouche C2 supporte la face d'application C21 pourvue des micro-aiguilles C211. La face d'application C21 est reliée à une tige ou tringle de transmission de vibrations C22 destinée à être couplée à l'organe de transmission de vibration I25 du module intermédiaire I2. Le corps C20 définit également une sortie de produit fluide C210 qui est située à proximité de la face d'application C21. Cette sortie de produit fluide C210 est raccordée par un conduit interne à un pointeau de perçage C203. Le module cartouche C2 comprend également un barillet rotatif C23 qui est monté rotatif sur ou dans le corps C20. Ce barillet C23 peut être entraîné en rotation par l'utilisateur au niveau de sa périphérie externe accessible. Le barillet C23 contient plusieurs réservoirs C24, qui peuvent se présenter sous la forme de poches écrasables ou de réservoirs à piston. Chaque réservoir C24 comprend une paroi frontale perçable 241 destinée à être percée par le pointeau de perçage C203. En position initiale, la membrane C241 est intacte et disposée de manière adjacente au pointeau de perçage C203, comme on peut le voir sur la figure 8. Dans cette position initiale de départ, la platine de poussée I221 de la tige d'actionnement I22 est disposée à proximité du fond du réservoir C24. Une poussée latérale sur l'organe d'actionnement I21 a pour effet de le déplacer, ce qui entraîne, par l'intermédiaire de la biellette I211, le déplacement axial de la tige d'actionnement I22, dont l'organe dans la platine de poussée I221 va premièrement déplacer le réservoir C23 dans son barillet pour percer la membrane 241, puis ensuite écraser le réservoir de manière à refouler son contenu jusqu'au niveau de la sortie de produit fluide 210. La figure 9a représente la position complètement enfoncée avec la platine de poussée I221 ayant atteint sa position avancée maximale. Le réservoir C24 est vide ou pratiquement vide.

Dès lors que l'utilisateur relâche sa pression sur l'organe d'actionnement I21, celui-ci retourne dans sa position initiale de départ sous l'action de la force du ressort de rappel I224. On se retrouve alors dans la configuration représentée sur la figure 9b. il suffit alors à l'utilisateur de faire tourner le barillet C23 pour amener un autre réservoir C24 en face de la platine de poussée I221 de la tige d'actionnement I22. Un cycle complet est alors atteint.

Dans ce mode de réalisation, le module cartouche C2 dans sa totalité peut être remplacé une fois que tous ses réservoirs C24 ont été vidés. En variante, il est également possible que seul le barillet C23 soit remplacé ou encore que seuls les réservoirs C24 soient remplacés en conservant le barillet C23. On voit ainsi que plusieurs formules sont possibles en conservant la même architecture pour le module cartouche C2.

On peut également envisager de mettre en œuvre le module cartouche C2 sur un module principal intégrant à la fois le module moteur M et le module intermédiaire I2. En d'autres termes, les moyens d'actionnement du module intermédiaire I2 seraient intégrés à un module commun ou principal intégrant tous les moyens pour actionner à la fois la face d'application C21 et les réservoirs C24.

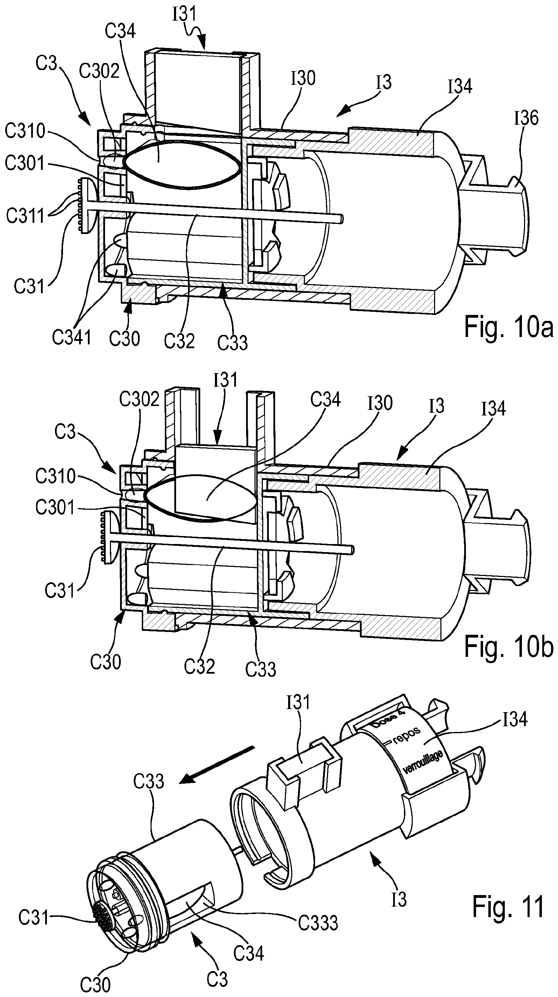

Les figures 10a, 10b et 11 représentent le troisième mode de réalisation de l'invention, dans lequel le module cartouche C3 intègre également un barillet rotatif C33 recevant plusieurs réservoirs C34. Tout comme dans le second mode de réalisation, l'ensemble du module cartouche C3 peut être remplacé, ou seulement son barillet C33 ou encore seulement ses réservoirs C34. Le module cartouche C3 comprend un corps fixe C30 qui forme la sortie de produit fluide C310, ainsi qu'une lame de coupe C301, dont la fonction sera donnée ci-après. La face d'application C31, avec ses micro-aiguilles C311, est positionnée à proximité directe de la sortie de produit fluide C310. Cette face d'application C31 se prolonge par une tige ou une tringle de transmission de vibrations C35 destinée à venir en prise avec les moyens de transmission de vibrations (non représentés) du module intermédiaire I3. Le barillet rotatif C33 est reçu de manière rotative sur ou dans le corps C30. Le barillet rotatif C33 comprend des fenêtres latérales C333 donnant accès aux réservoirs C34, qui comprennent un organe d'obturation amovible C341 qui fait saillie dans un conduit C302 débouchant au niveau de la sortie de produit fluide C310. Ainsi, lorsque le barillet C33 est entraîné en rotation, son organe d'obturation C341 vient en prise avec la lame de coupe C301 qui le sépare du reste du réservoir. Sur la figure 10a, on peut voir un organe d'obturation amovible 341 tombé en bas du corps C30. Cet organe d'obturation C341 est celui du réservoir C34 disposé en face du conduit C302. On comprend également que le réservoir disposé en dessous de C34 a encore son organe d'obturation amovible C341 pointant dans le corps C30. On peut ainsi dire que la lame de coupe C301 fait fonction d'organe d'ouverture du réservoir C34 par rotation du barillet C33. Avantageusement, les réservoirs C34 peuvent se présenter sous la forme de petites fioles écrasables en matière déformable.

Le module intermédiaire I3 comprend un corps I30, dans lequel est reçue une bague d'actionnement rotative I34 qui vient en prise avec le barillet C33, de manière à pouvoir l'entraîner en rotation. On voit sur la figure 11 que la bague d'actionnement I34 comprend des indications d'utilisation qui sont visibles à travers une fenêtre du corps I30.

Le module intermédiaire I3 comprend également un organe d'actionnement I31, qui se présente très simplement par un poussoir latéral que l'utilisateur peut enfoncer à l'aide d'un doigt. Ce poussoir latéral peut présenter la forme d'un plot dont le guidage est assuré par une cheminée formée par le corps I30. Lorsqu'un réservoir C34 est positionné en dessous de l'organe d'actionnement I31, son organe d'obturation amovible C341 a déjà été retiré par la lame C301. Cette configuration est représentée sur la figure 10a. Il suffit alors à l'utilisateur d'appuyer sur l'organe d'actionnement I31 pour venir en prise avec le réservoir souple C34 de manière à l'écraser. Cette configuration est représentée sur la figure 10b. Le produit fluide du réservoir C34 est ainsi refoulé à travers le conduit C302 jusqu'au niveau de la sortie de produit fluide C310. Lorsque l'utilisateur relâche sa poussée sur l'organe d'actionnement 131, celui-ci revient dans sa position de repos, soit par un ressort de rappel non représenté soit par la mémoire élastique du réservoir C34 qui revient à sa position initiale après vidage. Après application du produit fluide à l'aide de la face d'application 31 sur la peau de l'utilisateur, ce dernier peut faire tourner la bague d'actionnement I34 pour amener le prochain réservoir en dessous de l'organe d'actionnement I31 et répéter l'opération.

On peut remarquer que dans ce mode de réalisation le module cartouche C3 est pratiquement entièrement reçu à l'intérieur du module intermédiaire I3, seul son corps C30 en faisant saillie vers l'avant.

Bien que non représenté, on peut imaginer que le module intermédiaire I3 comprenne une trappe d'accès permettant d'accéder aux fenêtres latérales C333 du barillet pour retirer et insérer des réservoirs C34.

Le module intermédiaire I3 comprend des moyens de connexion I36 adaptés à venir en prise avec le module moteur M.

Dans les trois modes de réalisation qui viennent d'être décrits, l'applicateur à micro-aiguilles de l'invention comprend trois modules ou étages distincts qui sont connectés les uns aux autres, de préférence de manière amovible. Le module intermédiaire I1, I2, I3 comprend toujours un organe d'actionnement 111, I21, I31 et des moyens de transmission de vibrations I15, I25. Le module cartouche C1, C2, C3 comprend toujours une face d'application C11, C21, C31 dotée de micro-aiguilles C111, C211, C311, ainsi qu'une sortie de produit fluide C110, C210, C310. Il comprend également un organe de transmission de vibrations permettant de relier la face d'application aux moyens de transmission de vibrations du module intermédiaire.

Bien que certaines caractéristiques aient été décrites en relation avec cette architecture à trois étages au module, il faut bien comprendre que ces caractéristiques pourraient être mises en œuvre dans des architectures différentes. En particulier, les moyens d'actionnement du premier mode de réalisation pourraient être intégrés dans n'importe quel applicateur à micro-aiguilles, qu'il soit constitué d'un, de deux ou de trois étages ou modules. De même, le barillet rotatif C23, C33 a été décrit en relation avec cette architecture à trois étages ou modules. Cependant, ces barillets pourraient également être mis en œuvre dans n'importe quel applicateur à micro-aiguilles, qu'il comprenne trois étages, deux étages ou seulement un seul étage.

L'architecture à trois étages au module de l'invention a pour but principal de pouvoir adapter une grande variété de modules cartouche à un module moteur standard, grâce à l'adaptabilité et la modularité de l'étage intermédiaire.

## Revendications

1. Applicateur à micro-aiguilles pour appliquer un produit fluide sur la peau et le faire pénétrer dans la peau, l'applicateur définissant un axe longitudinal X et comprenant :
- une face d'application (C11 ; C21 ; C31) pourvue d'au moins une sortie de produit fluide (C110 ; C210 ; C310) et de plusieurs micro-aiguilles (C111 ; C211 ; C311),
- un moteur (M1) pour faire vibrer les micro-aiguilles (C111 ; C211 ; C311),
- au moins un réservoir de produit fluide (C13 ; C24 ; C34) relié à la sortie de produit fluide (C110 ; C210 ; C310),
- un organe d'actionnement (I11 ; I21 ; I31) pour acheminer le produit fluide du réservoir de produit fluide (C13 ; C24 ; C34) à la sortie de produit fluide (C110 ; C210 ; C310),
**caractérisé en ce qu'**il comprend trois modules distincts (M, C1 ; C2 ; C3, I1 ; I2 ; I3) raccordés axialement l'un à l'autre, à savoir :
- un module moteur (M) abritant le moteur (M1) et des accessoires pour faire fonctionner le moteur (M1),
- un module cartouche (C1 ; C2 ; C3) abritant ledit au moins un réservoir de produit fluide (C13 ; C24 ; C34) et formant la face d'application (C11 ; C21 ; C31),
- un module intermédiaire (I1 ; I2 ; I3) raccordé au module moteur (M) et raccordé de manière amovible au module cartouche (C1 ; C2 ; C3), le module intermédiaire (I1 ; I2 ; I3) comprenant l'organe d'actionnement (I11 ; I21 ; I31) et des moyens de transmission (I15 ; I25) pour transmettre les vibrations générées par le moteur (M1) du module moteur (M) aux micro-aiguilles (C111 ; C211 ; C311) du module cartouche (C1 ; C2 ; C3).

2. Applicateur selon la revendication 1, dans lequel le module intermédiaire (I1 ; I2 ; I3) est raccordé de manière amovible au module moteur (M).

3. Applicateur selon la revendication 1 ou 2, dans lequel le réservoir de produit fluide (C13 ; C24 ; C34) est à volume variable, le déplacement de l'organe d'actionnement (I11 ; I21 ; I31) engendrant une diminution de volume du réservoir de produit fluide (C13 ; C24 ; C34), de sorte qu'au moins une partie de son contenu est refoulée vers la sortie de produit fluide (C110 ; C210 ; C310).

4. Applicateur selon l'une quelconque des revendications précédentes, dans lequel le réservoir de produit fluide (C13 ; C24 ; C34) comprend une paroi mobile (C14) ou déformable, qui est déplacée en réponse à l'actionnement de l'organe d'actionnement (I11 ; I21 ; I31) selon l'axe longitudinal X ou transversalement à l'axe longitudinal X.

5. Applicateur selon l'une quelconque des revendications précédentes, présentant une configuration générale de stylo, adapté à être saisi entre le pouce et le majeur avec l'organe d'actionnement (I11 ; I21 ; I31) actionnable au moyen de l'index.

6. Applicateur selon l'une quelconque des revendications précédentes, dans lequel le module cartouche (C1 ; C2 ; C3) est raccordé de manière amovible au module intermédiaire (I1 ; I2 ; I3) par une connexion combinant un mouvement axial et un mouvement rotatif, tel qu'un vissage ou une baïonnette.

7. Applicateur selon l'une quelconque des revendications précédentes, dans lequel l'organe d'actionnement (111) comprend un poussoir latéral qui déplace axialement une tige d'actionnement (I12) qui agit progressivement sur le réservoir (C13) pour refouler des doses successives de produit fluide vers la sortie de produit fluide (C110).

8. Applicateur selon la revendication 7, dans lequel la tige d'actionnement (I12) est déplaçable axialement à l'encontre d'un ressort de rappel (I124) à partir d'une position de départ rétractée vers plusieurs positions avancées successives, dans lesquelles elle est bloquée en position contre tout retour en arrière par un dispositif de crans d'arrêt (113).

9. Applicateur selon la revendication 8, dans lequel le dispositif de cran d'arrêt (I13) est pourvu de moyens de débrayage (I14 ; I14' ; I14") pour permettre au dispositif de crans d'arrêt (I13) de libérer la tige d'actionnement (I12), qui retourne alors élastiquement vers sa position de départ rétractée.

10. Applicateur selon la revendication 9, dans lequel les moyens de débrayage (I14' ; I14") sont contraints dans un état inopérant par le raccord du module cartouche (C1) sur le module intermédiaire (I1) et libérés élastiquement dans un état débrayé par la séparation du module cartouche (C1), les moyens de débrayage (I14' ; I14") comprenant avantageusement un organe d'engagement (I143' ; I143") qui est déplaçable à partir d'un état libre, correspondant à l'état débrayé, vers un état contraint, correspondant à l'état opérant, par une came (C151, C1852 ; C16) formée par le module cartouche (C1) et qui est déplaçable à partir de l'état contraint vers l'état libre par un ressort de rappel (), dès qu'il est désengagé de la came (C151, C1852 ; C16).

11. Applicateur selon la revendication 1 ou 2, dans lequel le module cartouche (C2 ; C3) comprend un barillet rotatif (C23 ; C33) supportant plusieurs réservoirs de produit fluide (C24 ; C34), qui peuvent ainsi être amenés à tour de rôle dans une position de vidage, le module intermédiaire (12 ; I3) comprend un organe d'actionnement (121 ; I31) qui agit sur le réservoir situé en position de vidage pour refouler son contenu vers la sortie de produit fluide (C210 ; C310).

12. Applicateur selon la revendication 11, dans lequel l'organe d'actionnement (121) déplace axialement une tige d'actionnement (122) à l'encontre d'un ressort de rappel (1224) à partir d'une position de départ vers une position étendue correspondant au vidage maximal du réservoir (C24).

13. Applicateur selon la revendication 12, dans lequel l'organe d'actionnement (I31) agit directement sur le réservoir (C34) à travers une fenêtre latérale (C333) du barillet (C33).

14. Applicateur selon la revendication 13, dans lequel le module intermédiaire (I3) comprend des moyens d'actionnement (I34) pour entrainer le barillet (C33) du module cartouche (C3) en rotation.

15. Applicateur selon la revendication 13 ou 14, dans lequel la face d'application (C31) du module cartouche (C3) est bloquée en rotation par le module intermédiaire (I3), chaque réservoir (C34) comprenant un organe d'obturation amovible (C341) qui est retiré avant son arrivée en position de vidage par un organe d'ouverture (C301) solidaire de la face d'application (C31).

## Patentansprüche

1. Applikator mit Mikronadeln zum Auftragen eines Fluidprodukts auf die Haut und Eindringen lassen in die Haut, wobei der Applikator eine Längsachse X definiert und umfasst:
- eine Applikationsfläche (C11; C21; C31), die mit zumindest einem Fluidproduktauslass (C110; C210; C310) und mehreren Mikronadeln (C111; C211; C311) versehen ist,
- einen Motor (M1), um die Mikronadeln (C111; C211; C311) in Schwingung zu versetzen,
- zumindest ein Fluidproduktreservoir (C13; C24; C34), das mit dem Fluidproduktauslass (C110; C210; C310) verbunden ist,
- ein Betätigungsorgan (I11; I21; I31) zum Weiterleiten des Fluidprodukts vom Fluidproduktreservoir (C13; C24; C34) zum Fluidproduktauslass (C110; C210; C310),
**dadurch gekennzeichnet, dass** er drei verschiedene Module (M, C1; C2; C3, I1; I2; I3) umfasst, die axial miteinander verbunden sind, nämlich:
- ein Motormodul (M), das den Motor (M1) und Zubehörteile zum Betreiben des Motors (M1) aufnimmt,
- ein Kartuschenmodul (C1; C2; C3), das das zumindest eine Fluidproduktreservoir (C13; C24; C34) aufnimmt und die Applikationsfläche (C11; C21; C31) bildet,
- ein Zwischenmodul (I1; I2; I3), das mit dem Motormodul (M) verbunden ist und lösbar mit dem Kartuschenmodul (C1; C2; C3) verbunden ist, wobei das Zwischenmodul (I1; I2; I3) das Betätigungsorgan (I11; I21; I31) und Übertragungsmittel (I15; I25) zum Übertragen von vom Motor (M1) des Motormoduls (M) erzeugten Schwingungen auf die Mikronadeln (C111; C211; C311) des Kartuschenmoduls (C1; C2; C3) umfasst.

2. Applikator nach Anspruch 1, wobei das Zwischenmodul (I1; I2; I3) lösbar mit dem Motormodul (M) verbunden ist.

3. Applikator nach Anspruch 1 oder 2, wobei das Fluidproduktreservoir (C13; C24; C34) ein variables Volumen hat, wobei die Verschiebung des Betätigungsorgans (I11; I21; I31) eine Verringerung des Volumens des Fluidproduktreservoirs (C13; C24; C34) bewirkt, so dass zumindest ein Teil seines Inhalts zum Fluidproduktauslass (C110; C210; C310) hin verdrängt wird.

4. Applikator nach einem der vorhergehenden Ansprüche, wobei das Fluidproduktreservoir (C13; C24; C34) eine bewegliche oder verformbare Wand (C14) umfasst, die als Reaktion auf die Betätigung des Betätigungsorgans (I11; I21; I31) entlang der Längsachse X oder quer zur Längsachse X verschoben wird.

5. Applikator nach einem der vorhergehenden Ansprüche mit einer Konfiguration in Form eines Schreibstifts, der dazu ausgelegt ist, zwischen Daumen und Mittelfinger ergriffen zu werden, wobei das Betätigungsorgan (I11; I21; I31) mit dem Zeigefinger betätigt werden kann.

6. Applikator nach einem der vorhergehenden Ansprüche, wobei das Kartuschenmodul (C1; C2; C3) durch eine Verbindung, die eine axiale Bewegung und eine Drehbewegung kombiniert, wie etwa eine Schraubverbindung oder ein Bajonettverschluss, lösbar mit dem Zwischenmodul (I1; I2; I3) verbunden ist.

7. Applikator nach einem der vorhergehenden Ansprüche, wobei das Betätigungsorgan (I11) einen seitlichen Schieber umfasst, der axial eine Betätigungsstange (I12) verschiebt, die fortschreitend auf das Reservoir (C13) einwirkt, um aufeinanderfolgende Dosen an Fluidprodukt zum Fluidproduktauslass (C110) hin zu verdrängen.

8. Applikator nach Anspruch 7, wobei die Betätigungsstange (I2) entgegen einer Rückstellfeder (I124) aus einer eingezogenen Ausgangsstellung in mehrere aufeinanderfolgende vorgeschobene Stellungen axial verschiebbar ist, in denen sie durch eine Rastvorrichtung (I13) gegen ein Zurückstellen gesichert ist.

9. Applikator nach Anspruch 8, wobei die Rastvorrichtung (I13) mit Ausrückmitteln (I14; I14'; I14") versehen ist, mit denen die Rastvorrichtung (I13) die Betätigungsstange (I12) freigeben kann, die dann federnd in ihre eingezogene Ausgangsstellung zurückkehrt.

10. Applikator nach Anspruch 9, wobei die Ausrückmittel (I14'; I14") durch die Verbindung des Kartuschenmoduls (C1) mit dem Zwischenmodul (I1) in einen Nicht-Betriebs-Zustand beaufschlagt und durch die Trennung des Kartuschenmoduls (C1) federnd in einen ausgerückten Zustand freigegeben werden, wobei die Ausrückmittel (I14'; I14") vorteilhafterweise ein Eingriffsglied (I143'; I143") aufweisen, das durch einen vom Kartuschenmodul (C1) gebildeten Nocken (C151, C1852; C16) aus einem dem ausgerückten Zustand entsprechenden freien Zustand in einen dem Betriebszustand entsprechenden beaufschlagten Zustand verschiebbar ist und das durch eine Rückstellfeder aus dem beaufschlagten Zustand in den freien Zustand verschiebbar ist, sobald es von dem Nocken (C151, C1852; C16) gelöst ist.

11. Applikator nach Anspruch 1 oder 2, wobei das Kartuschenmodul (C2; C3) einen drehbaren Zylinder (C23; C33) umfasst, der mehrere Fluidproduktreservoirs (C24; C34) abstützt, die somit nacheinander in eine Entleerungsstellung gebracht werden können, wobei das Zwischenmodul (I2; I3) ein Betätigungsorgan (I21; I31) umfasst, das auf das in der Entleerungsstellung befindliche Reservoir einwirkt, um seinen Inhalt zum Fluidproduktauslass (C210; C310) hin zu verdrängen.

12. Applikator nach Anspruch 11, wobei das Betätigungsorgan (I21) eine Betätigungsstange (I22) entgegen einer Rückstellfeder (I224) axial aus einer Ausgangsstellung in eine ausgefahrene Stellung verschiebt, die der maximalen Entleerung des Reservoirs (C24) entspricht.

13. Applikator nach Anspruch 12, wobei das Betätigungsorgan (I31) durch ein Seitenfenster (C333) des Zylinders (C33) direkt auf das Reservoir (C34) einwirkt.

14. Applikator nach Anspruch 13, wobei das Zwischenmodul (I3) Betätigungsmittel (I34) zum Verdrehen des Zylinders (C33) des Kartuschenmoduls (C3) umfasst.

15. Applikator nach Anspruch 13 oder 14, wobei die Applikationsfläche (C31) des Kartuschenmoduls (C3) durch das Zwischenmodul (I3) gegen Drehung gesichert ist, wobei jedes Reservoir (C34) ein lösbares Verschlussorgan (C341) umfasst, das vor Erreichen der Entleerungsstellung durch ein fest mit der Applikationsfläche (C31) verbundenes Öffnungsorgan (C301) gelöst wird.

## Claims

1. Microneedle applicator for applying a fluid product on the skin and for making it penetrate into the skin, the applicator defining a longitudinal axis X and comprising:
- an application face (C11; C21; C31) provided with at least one fluid product outlet (C110; C210; C310) and with several microneedles (C111; C211; C311),
- a motor (M1) for making the microneedles (C111; C211; C311) vibrate,
- at least one fluid product reservoir (C13; C24; C34) connected to the fluid product outlet (C110; C210; C310),
- an actuation member (111; I21; I31) for conveying the fluid product from the fluid product reservoir (C13; C24; C34) to the fluid product outlet (C110; C210; C310),
**characterised in that** it comprises three distinct modules (M, C1; C2; C3, I1; I2; I3) axially connected to one another, namely:
- a motor module (M) housing the motor (M1) and accessories for making the motor (M1) function,
- a cartridge module (C1; C2; C3) housing said at least one fluid product reservoir (C13; C24; C34) and forming the application face (C11; C21; C31),
- an intermediate module (11; I2; I3) connected to the motor module (M) and removably connected to the cartridge module (C1; C2; C3), the intermediate module (11; I2; I3) comprising the actuation member (111; I21; I31) and transmission means (115; I25) for transmitting the vibrations generated by the motor (M1) of the motor module (M) to the microneedles (C111; C211; C311) of the cartridge module (C1; C2; C3).

2. Applicator according to claim 1, wherein the intermediate module (I1; I2; I3) is removably connected to the motor module (M).

3. Applicator according to claim 1 or 2, wherein the fluid product reservoir (C13; C24; C34) has a variable volume, the movement of the actuation member (111; I21; I31) leading to a decrease in volume of the fluid product reservoir (C13; C24; C34), such that at least some of its content is repelled to the fluid product outlet (C110; C210; C310).

4. Applicator according to any one of the preceding claims, wherein the fluid product reservoir (C13; C24; C34) comprises a mobile or deformable wall (C14), which is moved in response to the actuation of the actuation member (111; I21; I31) about the longitudinal axis X or transversally to the longitudinal axis X.

5. Applicator according to any one of the preceding claims, having a general pen configuration, adapted to being held between the thumb and the middle finger with the actuation member (111; I21; I31) which can be actuated by means of the index finger.

6. Applicator according to any one of the preceding claims, wherein the cartridge module (C1; C2; C3) is removably connected to the intermediate module (11; I2; I3) by a connection combining an axial motion and a rotary motion, such as a screwing or a bayonet.

7. Applicator according to any one of the preceding claims, wherein the actuation member (111) comprises a lateral pushbutton which axially moves an actuation rod (112) which acts progressively on the reservoir (C13) to repel successive fluid product doses to the fluid product outlet (C110).

8. Applicator according to claim 7, wherein the actuation rod (112) can be axially moved against a counter spring (1124) from a retracted starting position to several successive advanced position, in which it is blocked in position against any backward return by a catch device (113).

9. Applicator according to claim 8, wherein the catch device (113) is provided with disengagement means (114; I14'; I14") to allow the catch device (I13) to release the actuation rod (112), which thus elastically returns to its retracted starting position.

10. Applicator according to claim 9, wherein the disengagement means (114'; I14") are constrained in an inoperative state by the connector of the cartridge module (C1) on the intermediate module (11) and elastically released in a disengaged state by the separation of the cartridge module (C1), the disengagement means (114'; I14") advantageously comprising an engagement member (1143'; I143") which can be moved from a free state, corresponding to the disengaged state, to a constrained state, corresponding to the operative state, by a cam (C151, C1852; C16) formed by the cartridge module (C1) and which can be moved from the constrained state to the free state by a counter spring (), as soon as it is disengaged from the cam (C151, C1852; C16).

11. Applicator according to claim 1 or 2, wherein the cartridge module (C2; C3) comprises a rotary cylinder (C23; C33) supporting several fluid product reservoirs (C24; C34), which can thus be brought, in turn, into an emptying position, the intermediate module (12; I3) comprises an actuation member (121; I31) which acts on the reservoir located in the emptying position to repel its content to the fluid product outlet (C210; C310).

12. Applicator according to claim 11, wherein the actuation member (121) axially moves an actuation rod (122) against a counter spring (1224) from a starting position to an extended position corresponding to the maximum emptying of the reservoir (C24).

13. Applicator according to claim 12, wherein the actuation member (131) acts directly on the reservoir (C34) through a lateral window (C333) of the cylinder (C33).

14. Applicator according to claim 13, wherein the intermediate module (13) comprises actuation means (134) to rotate the cylinder (C33) of the cartridge module (C3).

15. Applicator according to claim 13 or 14, wherein the application face (C31) of the cartridge module (C3) is blocked in rotation by the intermediate module (13), each reservoir (C34) comprising a removable blocking member (C341) which is removed from its arrival in the emptying position by an opening member (C301) integral with the application face (C31).
